(19) Europäisches Patentamt
European Patent Office
Office européen des brevets

(11) Publication number: **0 503 710 A2**

(12) **EUROPEAN PATENT APPLICATION**

(21) Application number: **92200594.7**

(22) Date of filing: **02.03.92**

(51) Int. Cl.[5]: **A61K 37/02**, A61K 31/495, A61K 31/72, //(A61K31/72, 31:495,31:70,31:165,31:445), (A61K37/02,31:495,31:72)

(30) Priority: **08.03.91 EP 91200502**

(43) Date of publication of application: **16.09.92 Bulletin 92/38**

(84) Designated Contracting States: **AT BE CH DE DK ES FR GB GR IT LI LU NL PT SE**

(71) Applicant: **JANSSEN PHARMACEUTICA N.V.**
**Turnhoutseweg 30**
**B-2340 Beerse(BE)**

(72) Inventor: **Van Ginckel, Robert Franciscus**
**Maalderstraat 29**
**B-2290 Vorselaar(BE)**
Inventor: **Vanherck, Wim Victor Maria**
**Goorstraat 25**
**B-2470 Retie(BE)**

(74) Representative: **Wante, Dirk**
**Janssen Pharmaceutica N.V., Patent Department, Turnhoutseweg 30**
**B-2340 Beerse(BE)**

(54) **Flunarizine containing anti-neoplastic compositions.**

(57) Compositions containing flunarizine and an anti-neoplastic agent for anti-neoplastic therapy. Methods of treating neoplastic diseases comprising the co-administration of flunarizine and an anti-neoplastic agent and method of decreasing or eliminating developing or existing resistance to anti-neoplastic drug therapy.

EP 0 503 710 A2

## Background of the invention

Although a number of human cancers can now be treated with chemotherapeutic agents either in monotherapy or in multiple drug therapy, the result thereof often leaves much to be desired. Moreover, the successful outcome of cancer chemotherapy remains elusive for the majority of metastatic cancers.

One of the major problems in cancer chemotherapy is the development of resistance to cytotoxic drugs. Patients who did respond to a first course of chemotherapy frequently relapse because tumour cells seem to develop resistance against chemotherapeutic agents.

Further there exists a group of tumours which do not respond to chemotherapy and therefore can be considered to possess an intrinsic resistance thereto. Such tumours in particular are those in the epithelia, e.g. tumours of the colon, lungs, kidneys or melanoma.

A number of agents have been proposed which would overcome the problem of resistance to chemotherapeutic agents. However, such agents either only work in vitro or only work at too high doses to be of practical use, i.e. the side-effects become prominent.

It now has been found that the compound generically known as "flunarizine" decreases or even blocks the development of resistance to cytotoxic drugs on the one hand, and causes chemotherapeutic drugs to be effective against tumours with an intrinsic resistance to chemotherapy.

Compared with previous agents proposed to overcome chemotherapeutic drug resistance, flunarizine is quite potent and is active in vivo, has very little or no toxic side effects and has a larger therapeutic margin.

## Description of the invention

The present invention is concerned with pharmaceutical compositions comprising flunarizine and an anti-neoplastic agent, both active ingredients being present in an amount effective to exert an anti-neoplastic effect.

Flunarizine is the generic name of 1-[bis(4-fluorophenyl)methyl]-4-(3-phenyl-2-propenyl)piperazine and has been described in US-3,773,939. The structure of this compound can be represented as follows:

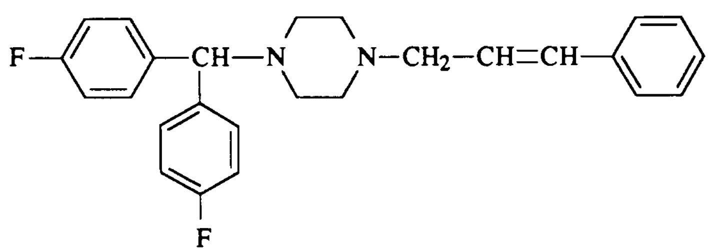

(I)

Flunarizine is a calcium entry blocker and is used as an anti-histaminic and particularly is effective in the treatment of cerebral and/or vascular insufficiency, vertigo and motion sickness.

The term flunarizine, as used hereinabove and hereinafter, is meant to comprise the base form as well as the pharmaceutically acceptable acid-addition salts thereof. The latter comprise those salts obtained by treatment of the base form with an appropriate acid such as, for example, inorganic acids such as hydrohalic acids, e.g. hydrochloric or hydrobromic acid; sulfuric acid; nitric acid; phosphoric acid and the like; or organic acids such as, for example, acetic, propanoic, hydroxyacetic, 2-hydroxypropanoic, 2-oxo-propanoic, ethanedioic, propanedioic, butanedioic, (Z)-butenedioic, (E)-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methyl-benzenesulfonic, cyclohexanesulfamic, 2-hydroxybenzoic, 4-amino-2-hydroxybenzoic and the like acids. A particularly interesting acid addition salt form of flunarizine is the dihydrochloride salt.

The term anti-neoplastic drug or agent refers to those active ingredients active against neoplasms and neoplastic diseases, such agents also being referred to as cytostatics, cytotoxic or cell-toxic drugs or agents, chemotherapeutics, anti-tumor agents, and the like terms. Generally, such agents are considered as alkylating agents, such as nitrogen mustards, ethyleneimine compounds, alkyl sulphonates, nitroureas; or as antimetabolites, such as certain types of antibiotics and plant alkaloids, e.g. vinca alkaloids; although other types of anti-neoplastic agents also exist. As examples of anti-neoplastic drugs which may be used according to the present invention, there may be mentioned actinomycins, ancitabine (cycloxytidine), aminoglutethimide, anisacride, azathioprine,bleomycins, busulfan, calusterone, carboplatin, carboquone, carmustine, chlorambucil, cisplatin, cyclophosphamide, cytarabine, dacarbazine, dactinomycin,

daunorubicin, doxorubicin (adriamycin), dromostanolone propionate, epirubicin, epitiostanol (epithioadrostanol), erbulozole, estramustine phosphate, etoposide, fluorouracil, diethylstilbestrol diphosphate, hydroxyurea, lomustine, melengestrol, melphalan, 6-mercaptopurine, methotrexate, mitobronitol, mitomycin C, mitopodozide, mitotane, mycophenolic acid, nimustine, pipobroman, piposulfan, prednimustine, procarbazine, razoxane, tegafur, teniposide, testolactone, tubulozole, triethylenethiophosphoramide, thioguanine, triazequone,trophosphamide, uramustine, vinblastine, vincristine, vindesine and the like anti-neoplastic drugs. Preferably, said agents are of the indol alkaloid type, the tetracycline or anthracycline type, the lignane type (derivatives of podophyllotoxin), or the cyclopeptide type.

Particular such drugs are vincristine, vinblastine, actinomycin D and F1, gramicidin, colchicine, etoposide, teniposide, adriamycine. The term anti-neoplastic drugs is meant to also comprise the possible pharmaceutically acceptable acid-addition or base-addition salts, and the possible stereoisomeric forms of the aforementioned agents.

In a further aspect, the present invention provides a method of treating neoplastic diseases in patients suffering therefrom or avoiding the growth of neoplasms in patients where such growth likely is to take place, said method comprising the administration of an effective amount of flunarizine and an anti-neoplastic agent. Or, the present invention concerns the use of flunarizine for the manufacture of a medicament for treating neoplastic diseases in patients suffering therefrom or avoiding the growth of neoplasms in patients where such growth is likely to take place.

Hence, flunarizine and the anti-neoplastic agent also can be administered prophylactically, e.g.in patients having previously suffered from a neoplastic disease and being cured.

In still a further aspect, the present invention provides a method of decreasing or eliminating a developing or existing resistance to anti-neoplastic drug therapy, or avoiding such resistance from arising, in patients suffering from neoplastic diseases, or likely to develop neoplastic diseases, said method comprising the administration to said patients of an effective amount of flunarizine. Or, the present invention concerns the use of flunarizine for the manufacture of a medicament for decreasing or eliminating a developing or existing resistance to anti-neoplastic drug therapy, or avoiding such resistance from arising.

As used hereinabove, the expression neoplastic diseases on the one hand refers to diseases caused by neoplasms (or tumours) with an intrinsic resistance to chemotherapy, and on the other hand, diseases caused by neoplasms which can develop, are developing or have developed a resistance to anti-neoplastic therapy.

The active ingredient flunarizine may be administered before, during or after the administration of the anti-neoplastic agent, provided that the time between the administration of flunarizine and the administration of the anti-neoplastic agent is such that flunarizine is allowed to influence the activity of the anti-neoplastic agent in such a way that an adequate anti-neoplastic effect is exerted. When simultaneous administration of flunarizine and an anti-neoplastic agent is envisaged, a composition containing both an anti-neoplastic agent and flunarizine may be particularly convenient. Or, flunarizine and the anti-neoplastic agent may be administered separately in the form of suitable compositions, which can be prepared as described hereinafter. The form of such compositions will evidently depend on the kind of anti-neoplastic drug. If the anti-neoplastic drug is preferably administered parenterally, e.g. intravenously, the combination composition evidently will have to be suitable for parenteral administration. In the latter instance the composition will take the form of aqueous solutions containing flunarizine and the anti-neoplastic agent, and hence the flunarizine active ingredient will have to be brought in a form suitable for making such aqueous solutions, e.g. by formulating it as a salt form, such as the hydrochloride salt form, or formulated in a suitable carrier, e.g. a cyclodextrin (CD) or in particular a cyclodextrin derivative such as the cyclodextrin derivates described in US-3,459,731, EP-A-149,197 or EP-A-197,571. Typically such derivatives comprise $\alpha$-, $\beta$- or $\gamma$-CD wherein one or more hydroxylgroups are substituted with $C_{1-6}$alykl, particularly methyl, ethyl or isopropyl; hydroxy-$C_{1-6}$alkyl, particularly hydroxyethyl, hydroxypropyl or hydroxy-butyl; carboxy-$C_{1-6}$alkyl, particularly carboxymethyl or carboxyethyl; $C_{1-6}$alkyloxy-carbonyl$C_{1-6}$alkyl or carboxy$C_{1-6}$alkyloxy$C_{1-6}$alkyl, particularly carboxymethoxypropyl or carboxyethoxypropyl. Especially noteworthy as complexants and/or solubilizers are $\beta$-CD, 2,6-dimethyl-$\beta$-CD and in particular 2-hydroxypropyl-$\beta$-CD, 2-hydroxyethyl-$\beta$-CD, 2-hydroxyethyl-$\gamma$-CD, 2-hydroxypropyl-$\gamma$-CD and (2-carboxymethoxy)propyl-$\beta$-CD. In the aforementioned cyclodextrin derivatives, the DS (degree of substitution, i.e. the average number of substituted hydroxy functions per glucose unit) preferably is in the range of 0.125 to 3, in particular 0.2 to 2, or 0.2 to 1.5. More preferably the DS ranges from about 0.2 to about 0.7, in particular from about 0.35 to about 0.5 and most particularly is about 0.4. The MS (molar degree of substitution, i.e. the average number of moles of the substituting agent per glucose unit) is in the range of 0.125 to 10, in particular of 0.3 to 3, or 0.3 to 1.5. More preferably the MS ranges from about 0.3 to about 0.8, in particular from about 0.35 to about 0.5 and most particularly is

about 0.4.

In case of separate administration, the anti-neoplastic drug may e.g. be administered intravenously and flunarizine orally. Or, flunarizine may first be administered intravenously and subsequently orally e.g. to first build up a certain blood level and subsequently to maintain said level. Oral dosage forms of flunarizine may be the normally used forms, e.g. capsules, but also the formulations described in EP-A-292,050.

In the compositions containing both flunarizine and an anti-neoplastic agent, the amount of the latter will be largely dependent upon the nature of said agent. Said amount will evidently be such that a suitable anti-neoplastic therapeutic effect is obtained. The anti-neoplastic agents for use in the compositions of the present invention differ both in molecular weight and in activity, and therefore may be present in fairly different quantities. Depending on the kind of anti-neoplastic agent the amount of said agent will be dosed such that the dose administered ranges from 0.1 to 10 mg/kg or from 0.5 to 5 mg/kg or from 1 to 2.5 mg/kg per unitary dose for the more active agents having a lower molecular weight and multiples of said doses for less active agents and/or having a high molecular weight.

In general, the anti-neoplastic agents will be administered in the so-called subtoxic or maximal tolerable dose, i.e. the maximal dose which does not give rise to the side-effects or toxic effects becoming harmful or problematic. In the specific case of adriamycine said dose is about 2.5 mg/kg, in case of other anti-neoplastic agents said dose may be different, depending on the agent, for example 1 mg/kg.

Flunarizine in said compositions will be dosed such that the dose administered ranges from 1 to 160 mg/kg body weight, in particular from 2.5 to 40 mg/kg and more in particular from 2.5 to 10 mg/kg. The dose of flunarizine and the anti-neoplastic agent in said compositions therefore will vary upon the kind of patient to be treated, e.g. adult or child, and upon the dose regimen envisaged by the clinician treating the patient, e.g. once daily, or twice, three or more times daily, each administration comprising one, two or more unitary doses. The amount of flunarizine in the compositions of the present invention may therefore vary, and may for example be in the range from 1 to 200 mg, or from 1 to 100, or 1 to 50 mg per dosage unit form. The same applies for the dose of the anti-neoplastic agent, which, for example, may be 0.5 to 10 mg, 10 to 100 mg or 100 to 500 mg per unitary dose, depending on the kind of anti-neoplastic agent.

The present invention also comprises products containing flunarizine and an anti-neoplastic agent as a preparation for simultaneous, separate or sequential use in anti-neoplastic therapy. Such products may comprise, for example, a kit comprising a container with a suitable composition containing flunarizine and another container containing a composition with an anti-neoplastic agent. Such a product may have the advantage that a physician wishing to apply anti-neoplastic therapy, can select on the basis of the diagnosis of the patient to be treated, the appropriate amounts of each component and the sequence and timing of the administration thereof.

To prepare the pharmaceutical compositions of the present invention, the active ingredient or ingredients are intimately mixed with a suitable pharmaceutical carrier. Said pharmaceutical compositions are desirably in unitary dosage form suitable, preferably, for administration orally, rectally or by parenteral injection. For example, in preparing the compositions in oral dosage form, any of the usual pharmaceutical carriers may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. As mentioned before, in case of parenteral formulations, the active ingredient flunarizine preferably will be formulated in cyclodextrins, in particular in the aforementioned substituted cyclodextrins. Such compositions are prepared in the usual manner, e.g. as described in EP-A-149,197 or EP-A-197,571. For example, said compositions can conveniently be prepared by dissolving the cyclodextrin or ether derivative thereof in water and adding thereto the compound of formula (I) as well as other adjuvants and components such as, for example, sodium chloride, potassium nitrate, glucose, mannitol, sorbitol, xylitol and buffers such as, for example, phosphate, acetate or citrate buffers. The amount of the cyclodextrin or ether derivative thereof in the final composition generally ranges from about 1% to about 40%, particularly form 2.5% to 25% and more particularly from 5 % to 20%.

It is especially advantageous to formulate the aforementioned pharmaceutical compositions in dosage unit form for ease of administration and uniformity of dosage. The term dosage unit form refers to physically discrete units suitable as unitary dosages, each unit containing a predetermined quantity of the

active ingredient or ingredients, calculated to produce the desired therapeutic effect, in association with the required pharmaceutical carrier. Examples of such dosage unit forms are tablets (including scored or coated tablets), capsules, pills, injectable solutions or suspensions.

## Experimental part

### A. Composition examples

The following formulations exemplify typical pharmaceutical compositions in dosage unit form suitable for administration to warm-blooded animals in accordance with the present invention.

"Active ingredient" (A.I.) as used throughout these examples relates to a compound of formula (I) or a pharmaceutically acceptable acid addition salt thereof, or to an anti-neoplastic agent, or to a combination of both flunarizine and an anti-neoplastic agent.

#### Example 1: Oral drops

500 g of the A.I. is dissolved in 0.51 of 2-hydroxypropanoic acid and 1.51 of the polyethylene glycol at 60~80 °C. After cooling to 30~40 °C there are added 351 of polyethylene glycol and the mixture is stirred well. Then there is added a solution of 1750 g of sodium saccharin in 2.51 of purified water and while stirring there are added 2.51 of cocoa flavor and polyethylene glycol q.s. to a volume of 501, providing an oral drop solution comprising 10mg/ml of the A.I. The resulting solution is filled into suitable containers.

#### Example 2 : Oral solutions

9 g of methyl 4-hydroxybenzoate and 1 g of propyl 4-hydroxybenzoate are dissolved in 4 l of boiling purified water. In 3 l of this solution are dissolved first 10 g of 2,3-dihydroxybutanedioic acid and thereafter 20 g of the A.I. The latter solution is combined with the remaining part of the former solution and 121 of 1,2,3-propanetriol and 3 l of sorbitol 70% solution are added thereto. 40 g of sodium saccharin are dissolved in 0.51 of water and 2 ml of raspberry and 2 ml of gooseberry essence are added. The latter solution is combined with the former, water is added q.s. to a volume of 20 l providing an oral solution comprising 5 mg of the A.I. per teaspoonful (5 ml). The resulting solution is filled in suitable containers.

#### Example 3 : Capsules

20 g of the A.I., 6 g sodium lauryl sulfate, 56 g starch, 56 g lactose, 0.8 g colloidal silicon dioxide, and 1.2 g magnesium stearate are vigorously stirred together. The resulting mixture is subsequently filled into 1000 suitable hardened gelatin capsules, each comprising 20 mg of the A.I..

#### Example 4 : Film-coated tablets

##### Preparation of tablet core

A mixture of 100 g of the A.I., 570 g lactose and 200 g starch is mixed well and thereafter humidified with a solution of 5 g sodium dodecyl sulfate and 10 g polyvinylpyrrolidone (Kollidon-K 90®) in about 200 ml of water. The wet powder mixture is sieved, dried and sieved again. Then there are added 100g microcrystalline cellulose (Avicel®) and 15 g hydrogenated vegetable oil (Sterotex ®). The whole is mixed well and compressed into tablets, giving 10.000 tablets, each comprising 10 mg of the active ingredient.

##### Coating

To a solution of 10 g methyl cellulose (Methocel 60 HG®) in 75 ml of denaturated ethanol there is added a solution of 5 g of ethyl cellulose (Ethocel 22 cps®) in 150 ml of dichloromethane. Then there are added 75 ml of dichloromethane and 2.5 ml 1,2,3-propanetriol. 10 g of polyethylene glycol is molten and dissolved in 75 ml of dichloromethane. The latter solution is added to the former and then there are added 2.5 g of magnesium octadecanoate, 5 g of polyvinylpyrrolidone and 30 ml of concentrated colour suspension (Opaspray K-1-2109®) and the whole is homogenated. The tablet cores are coated with the thus obtained mixture in a coating apparatus.

Example 5 : Injectable solutions

a) 1.8 g methyl 4-hydroxybenzoate and 0.2 g propyl 4-hydroxybenzoate are dissolved in about 0.51 of boiling water for injection. After cooling to about 50 ° C there are added while stirring 4 g lactic acid, 0.05 g propylene glycol and 4 g of the A.I.. The solution is cooled to room temperature and supplemented with water for injection q.s. ad 11 volume, giving a solution of 4 mg A.I. per ml. The solution is sterilized by filtration (U.S.P. XVIIp. 811) and filled in sterile containers.

b) An injectable solution comprising a cyclodextrin can be prepared with the following composition:

| | |
|---|---|
| Active ingredient | 5.9 mg |
| Hydroxypropyl-β-cyclodextrin (MS = 0.4) | 100 mg |
| Sodium chloride | 4.9 mg |
| Sodium hydroxide | q.s. ad pH 4.5 |
| Water | q.s. ad 1 ml |

Example 6 : Suppositories

3 g A.I. is dissolved in a solution of 3 g 2,3-dihydroxybutanedioic acid in 25 ml polyethylene glycol 400. 12 g surfactant (SPAN®) and triglycerides (Witepsol 555®) q.s. ad 300 g are molten together. The latter mixture is mixed well with the former solution. The thus obtained mixture is poured into moulds at a temperature of 37-38 ° C to form 100 suppositories each containing 30 mg of the A.I.

B. Pharmacological example

The potential of flunarizine as a potent enhancer of adriamycine (ADR) cytotoxicity in the murine sarcoma MO4 (semi-resistant cell line) "in vitro" and "in vivo" and its reversed adriamycine resistance in the P388/ADR (adriamycine resistant cell line) murine leukemia "in vivo"can be demostrated by the following procedure.

Fragments of subcutaneously grown tumours were inserted under the renal capsule in mice and their initial size measured under a microscope. At day 7, the mice were killed and the final size of the implants determined. The change in tumor size was expressed as a percentage of the initial size, the latter considered to be 100%. Daily intraperitoneal treatment with ADR alone or in combination with flunarizine which was formulated in hydroxypropyl-β-cyclodextrin was installed from day 1 until day 6. Control animals received the vehicle hydroxypropyl-ß-cyclodextrin.

Flunarizine given as monotherapy had no effect; adriamycine displayed only marginal effect on the MO4-tumor (25% growth reduction with half the maximal tolerated dose 2.5 mg/kg; no effect with lower dosages) and was inactive in reducing the growth of P388/ADR. Combination therapy of flunarizine and adriamycine consistently reduced the growth of MO4 and P388/ADR in a dose-dependent manner. Flunarizine dosing with 40 mg/kg down to 2.5 mg/kg - in combination with different doses of adriamycine - further reduced the growth of MO4-implants with 40-60 % and adriamycine sensitivity in the P388/ADR was completely restored with flunarizine doses down to 10 mg/kg.

## Claims

**1.** A pharmaceutical composition containing a pharmaceutically acceptable carrier and as active ingredients an effective amount of flunarizine and an anti-neoplastic agent.

**2.** A composition according to claim 1 wherein the anti-neoplastic agent is vincristine, vinblastine, actinomycin D or F1, gramicidin, colchicine, etoposide, teniposide, or adriamycine.

**3.** A composition according to claim 1 or 2 further comprising a cyclodextrin or a derivative thereof.

**4.** A process for preparing a pharmaceutical composition as claimed in any one of claims 1 to 3 characterized in that an effective amount of flunarizine and an anti-neoplastic agent are intimately mixed with a pharmaceutically acceptable carrier.

**5.** A product containing flunarizine and an anti-neoplastic agent as a preparation for simultaneous, separate or sequential use in anti-neoplastic therapy.

**6.** A product according to claim 5 consisting of a kit which comprises a container with a suitable composition containing flunarizine and another container containing a composition with an anti-neoplastic agent.

**7.** The use of flunarizine for the manufacture of a medicament for treating neoplastic diseases in patients suffering therefrom or avoiding the growth of neoplasms in patients where such growth is likely to take place.

**8.** The use of flunarizine for the manufacture of a medicament for decreasing or eliminating a developing or existing resistance to anti-neoplastic drug therapy in patients suffering from neoplastic diseases, or avoiding such resistance from arising.